# EUROPEAN PATENT SPECIFICATION

(11) **EP 0 912 195 B1**
(45) Date of publication and mention of the grant of the patent: **21.01.2009**
(21) Application number: 97927484.2
(22) Date of filing: 24.06.1997
(51) Int. Cl.: A61K 39/385, A61K 39/00, A61K 39/23, C07K 14/015, C07K 7/23

(54) **VACCINE COMPRISING ANTIGENS BOUND TO CARRIERS THROUGH LABILE BONDS**
EIN DURCH LABILE BINDUNG AN EINEN TRÄGER GEBUNDENES ANTIGEN ENTHALTENDER IMPSTOFF
VACCINS COMPRENANT DES ANTIGENES FIXES SUR LEURS SUPPORTS PAR DES LIAISONS LABILES

(30) Priority: 25.06.1996 EP 96201766
(43) Date of publication of application: 06.05.1999
(73) Proprietor: PEPSCAN SYSTEMS B.V., 8219 PH Lelystad (NL)
(72) Inventor: BEEKMAN, Nico Johannes Christiaan Maria, NL-8014 GL Zwolle (NL); SCHAAPER, Wilhelmus Martinus Maria, NL-1313 NJ Almere (NL); DALSGAARD, Kristian, DK-4771 Kalvehave (DK); MELOEN, Robert Hans, NL-8231 AP Lelystad (NL)
(74) Representative: Prins, Adrianus Willem
(86) International application number: PCT/NL1997/000354
(87) International publication number: WO 1997/049425

(56) References cited:
- EP-A- 0 431 327
- EP-A- 0 491 077
- WO-A-90/11298
- WO-A-92/12995
- WO-A-93/02706
- WO-A-94/17098
- WO-A-94/25060
- WO-A-95/08348
- US-A- 5 256 641
- RICHARDSON J. ET AL: "Neutralization sensitivity and accesibility of continuous B cell epitopes of the feline immunodeficincy virus envelope" THE JOURNAL OF GENERAL VIROLOGY, vol. 77, April 1996, pages 759-777, XP002041747

## Description

The invention relates to the field of immunization and vaccines. By vaccination, antibody and/or cellular responses are elicited in the vaccinated animal or human. These responses are directed against the antigen or antigens that are used in the vaccine. It has long been known that the antigenicity and immunogenicity of antigens may vary, depending for example on the way the antigen is presented to the vaccinated animal, or on the size and structure of the antigen molecule.

A widely known way of increasing the immunogenicity of a vaccine preparation is the addition of adjuvants to this preparation. However, such adjuvants stimulate the immune response only in an aspecific way, and do not increase the specific antigenicity or immunogenicity of the antigen used. Enhancement of specific antigenicity or immunogenicity of antigens that are themselves poorly antigenic or immunogenic is often achieved by coupling the antigen to carrier compounds. Synthetic peptides have frequently been used to immunize animals. Peptides of less then about 20 to 30 amino acids are, however, notorious examples of such poor immunogens. To increase their specific antigenicity or immunogenicity it is assumed that the molecular weight needs to be increased.

Many attempts have been made to conjugate antigens in a stable bond to carrier molecules, using different carrier compounds such as the carrier proteins keyhole limpet haemocyanin (KLH) or ovalbumin (OVA), in order to increase the molecular weight. Such immunoconjugation may lead to very complex structures, and generate unwanted side-effects, in the sense that e.g. unwanted antibodies are elicited that are specifically directed against the carrier compound and the areas of the conjugated molecule where the linkage of the antigen with the carrier have taken place.

Other methods that have been applied are the coupling of fatty acid groups to proteins or peptides of low antigenicity or immunogenicity. The main purpose of the introduction of fatty acids into antigens has been to anchor hydrophillic antigens on adjuvant- and immuno-presentation systems, e.g. liposomes and iscoms, because such systems require the presence of hydrophobic molecules to allow their incorporation into these systems. Such procedures of vaccine preparation have been used with some success with model protein antigens and with less success with synthetic peptides. Acylation of the lysines of ovalbumine with palmitic acid was found to enhance the major histocompatibility complex (MHC) class II-restricted presentation. This suggests that conjugation with longer fatty acids would lead to the formation of lipopeptide T-cell epitopes with increased affinity for binding to MHC class II and/or T-cell receptors (20).

Such coupling of fatty acids has been achieved by acylation of proteins or peptides (26) intended for use in immunization/vaccination purposes, simply by linking the fatty acid covalently to the antigen. In these studies, predominantly palmitic acid or myristic acid have been used. For protein antigens, the introduction of lipid tails' (palmitylation) has been performed mainly by using palmitic acid N-hydroxysuccinimide, leading to an irreversible and stable bond between the protein and the fatty acid. For synthetic peptide antigens, the most common procedure is performed after synthesis of the peptide, by adding palmitic acid to the free amino group in the terminal amino acid by continuing peptide synthesis chemistry. This leads to an irreversible amide bond between the palmitic tail and the peptide.

European patent application EP-A-431327 discloses vaccines comprising T-cell epitope peptides linked to palmitic acid derivatives. The peptides are bound to the palmitoyl carrier through amide bonds.
International patent application WO-A-9508348 discloses methods for producing immunogenic constructs wherein at least one first carbohydrate-containing moiety is activated with a cyanylating reagent, where after the activated carbohydrate is covalently joined to a second moiety.
United States patent US 5,256,641 discloses methods for eliciting or inhibiting an immune response in an animal, for instance a human. Reagents are provided which comprise antigenically-active peptides covalently linked to polar lipid carrier molecules.

We have now found a procedure which, instead of coupling the antigen irreversibly to a carrier compound, allows for coupling between the antigen (be it protein or peptide or carbohydrate or any other molecule to be used as an antigen for immunization/vaccination procedures) and the carrier compound in a reversible and labile way, with a so-called labile-linking method. With a 'labile-link' a labile chemical bond between antigen and carrier protein is meant. Labile should be understood as either chemically or enzymatically labile. A chemically labile bond cleaves for example under conditions normally found in the body, e.g. at basic pH such as can be found in certain body tissues, as at acidic pH which can be found in other tissues or certain cell compartments, whereas an enzymatically labile bond cleaves in the presence of enzymes, such as thioesterases or esterases, which are present in bodily tissues.

Such cleavages result in a dissociation of the antigen and the carrier compound, after administration of the vaccine. In this way, as will be demonstrated in detail in the experimental part of this description, surprisingly a better immune response can be elicited by an in itself poorly immunogenic antigen than by methods that provide a stable link between the antigen and carrier compound.

The invention not only allows for the introduction of carrier compounds, such as lipid tails, into antigens but also allows for a better routing to the cell surface of antigen presenting cells, after which the antigen becomes dissociated and may be better processed by those antigen presenting cells, resulting in a much better immune response. Therefore, the invention is not simply aimed at anchoring antigens to carrier proteins, adjuvants, or other presentation systems, but is in its own right capable of greatly increasing the antigenicity or immunogenicity of antigens.

A theoretical explanation which should not be seen as restricting the invention is that antigens with reversible bonds or labile links to carrier compounds, for instance by palmitic acid acylation of thiol groups in peptides, would facilitate the routing of these antigens to various cell compartiments and enhance in this way their immunogenicity.

Peptides have been substituted with palmitic acid residues in quite a number of ways: by conjugation with Nα,Nε-dipalmitoyllysine peptides (8,5), with Nα-palmitoyl-S-(2,3-*bis*-palmitoloxy-(2RS)-propyl)-(R)-cysteine (Pam₃Cys-peptides (5,13)), palmitoylated polylysine (23,24), Pam₃Cys-Multiple Antigen Peptides (4,10), lipo-Multiple Antigen Peptides (9,10), N-palmitoylated proteins used for incorporation into ISCOMs (3,14,17), and peptidyl-Nε-palmitoyl-lysines (5). In these examples, palmitoylation was at an amino group or a hydroxyl group, providing irreversible bonds such as amides.

The present invention as exemplified in the experimental part, which utilizes the formation of a labile bond, now provides a very simple approach to obtain labily linked antigen and carrier compound via the controlled conjugation of the antigen with one long-chain fatty acid. The presence of the fatty acids increases the uptake of the antigen into antigen presenting cells, and, due to the labile or reversible nature of the bond, the antigen becomes separated again, and specific antibody formation directed to the antigen only is elicited.

In the experimental part the concept of labile linking is demonstrated with synthetic peptides as antigen linked via a thioester bond to palmitic acid as carrier compound or with a synthetic peptide as antigen linked via a disulfide bond to a N-palmitoyl peptide as carrier compound. A preferred part of the invention is thus illustrated by the examples provided. However, any vaccine or immunogenic preparation in wich the antigen is reversibly or instabily or labily bound to the carrier compound by whatever labile link imaginable with the purpose that the antigen dissociates from the carrier compound after administration of the vaccine is also part of the invention. For example, a vaccine composition comprising a glycopeptide, linked via a disulfide bridge to ovalbumine, will dissociate as soon as the composition encounters reducing circumstances, as for instance can occur in the blood in the presence of glutathion.

As antigen, other types of molecules to which an immune response preferably needs to be elicited, such as polypeptides, proteins with or without carbohydrate moieties or other side chains, carbohydrate chains themselves, nucleic acid molecules, haptens, etc., can be chosen. As carrier compound, other fatty acids can be used, but also carrier proteins, such as KLH or OVA, or fatty acid peptides, or other types of molecules can be fused as carrier compound.

For the labile link between the antigen and the carrier compound, the thioester bond illustrates a preferred part of the invention. However, other chemical bonds such as esters and disulfide bonds that are labile under physiological conditions (such as pH or osmolarity or salt concentrations or the presence of reducing agents or enzymes) which are usually present in body fluids or in or on body tissue or cells also fit the purpose of providing a vaccine or immunogenic preparation in which the antigen dissociates from its carrier compound under physiological conditions, i.e. after it has been administered. Typically good examples of labile linkers can be found in the field of tumortherapy where such linkers have been applied between antibodies and cytostatic agents.

Also, vaccines and immunogenic preparations that are prepared according to the invention can be mixed with carrier compounds and adjuvants that are traditionally used to render antigens more antigenic or immunogenic. In addition, vaccines and immunogenic preparations that are prepared according to the invention can be mixed with other compounds or adjuvants or antigen delivery systems that are specifically chosen to hasten or slow the rate of dissociation of the antigen from the carrier compound after the preparation has been administered, i.e. by surrounding the preparation with conditions in which the dissociation rate is altered, and thereby shielding it temporarily from normal physiological conditions. For sustained release of the antigen from the carrier it may also be useful to apply different linkers having different dissociation constants between carrier and antigen.

Furthermore, vaccines and immunogenic preparations that are prepared according to the invention can be administered in various ways known in the art, for instance by but not limited to intramuscular, intranasal, intraperitoneal, intradermal, intracutanous, mucosal or aerosol application of the preparation.

### EXAMPLES

### Introduction

The concept of labile linking is demonstrated with synthetic peptides as antigen and palmitic acid or a N-palmitoylpeptide as carrier compound.

Conditions for acylation of peptides in solution or on solid phase were established. Furthermore, it was demonstrated that the stability of the bond influences the immunological efficacy, because in our study conjugation of a fatty acid implies either formation of a stable amide when N-acylation is involved, or formation of a labile thioester in the case of S-acylation. It was found that this difference in site of acylation (N or S) is important with respect to its immunogenicity. Upon immunization it was found that labile S-palmitoylated peptides or peptides coupled to a N-palmitoylated peptide via a labile disulfide bridge are superior to N-palmitoylated peptides and at least comparable to KLH conjugated peptides with respect to response time of detectable antibody formation or biological effect. A theoretical explanation is that, under biological or physiological conditions, the presence of appropriate fatty acid chains chemically linked through labile thioester or disulfide bonds, improves immunogenicity, probably because it represents a favourable substrate for take up and processing in cells of the immune system. This processing can be mediated by palmitoyl thioesterases, that have been isolated (18), or for disulfides by glutathion.

Unlike amides and esters, thioesters are readily susceptible to nucleophilic attack. The chemical stability of palmitoyl thioesters had thus to be considered at physiological pH and during deprotection conditions (acid, acid-thiol, base). Nine peptide constructs (A, B, C, D, E, F, G, H and I, Fig. 1) were prepared as vaccine preparation, two peptides represent a GnRH-tandem peptide, five are based on the N-terminus of VP2 (positions cys-2-21) of canine parvovirus (CPV (11)), and two are from the V3 loop of feline immunodeficiency virus (FIV). They differ by their acylation sites (S, α-N or ε-N, Fig. 1). It was found that S-palmitoylated peptides induce high levels of antibodies, comparable to or slightly better than KLH-MBS linked peptides, and superior to N-palmitoylated peptides with respect to response time of detectable antibodies (CPV) or biological effect (GnRH).

### MATERIALS:

N-methylpyrrolidone (NMP), 2-(1H-benzotriazol-1-yl)-1,1,3,3,-tetramethyluronium hexafluorophosphate (HBTU) and piperidine were peptide synthesis grade and obtained from Perkin Elmer/ABI (Warrington, UK). Dicyclohexylcarbodiimide (DCC), dimethylformamide (DMF), N-hydroxybenzotriazole (HOBt), diisopropylethylamine (DIEA), acetonitrile (ACN), trifluoroacetic acid (TFA), thioanisole (TA), phenol, palmitic acid and ethanedithiol (EDT) were pro-analysis grade and were obtained from Merck (Darmstadt, Germany). Diethylether was purified over a column of activated basic aluminumoxide and DIEA was distilled twice over ninhydrin and potassiumhydroxide before use. Fmoc-amino acid derivatives and resin ( 4-(2',4'-dimethoxyphenyl-Fmoc-aminomethyl)phenoxy resin (Rink resin) (19)) were obtained from Saxon Biochemicals (Hannover, Germany). 4-methylbenzhydrylamine (MBHA) resin was obtained from Novabiochem, Laufelfingen, Switzerland. For analytical HPLC we used two Waters pumps model 510, a Waters gradient controller model 680, a Waters WISP 712 autoinjector, and a Waters 991 photodiode array detector. Products were analyzed in a linear gradient from water with 0.1% TFA to 60% acetonitrile/water with 0.1% TFA in 60 minutes on a Waters Delta Pak C₁₈-100 (3.9x150mm, 5µm) column at 1 ml/min.

Amino acid analysis was performed using a Waters Pico-Tag system, after hydrolysis in a Pico-Tag workstation using 6N HCl at 150°C for 1 hour, and derivatization with phenylisothiocyanate. Preparative HPLC was carried out using a Waters Prep 4000 liquid chromatograph, equipped with a Waters RCM module with two PrepPak cardriges plus guard cardridge (40x210 mm or 25x210 mm) filled with Delta-Pak C₁₈-100 (15 µm) material. Peptides were detected at 230 nm using a Waters 486 spectrophotometer with a preparative cell.

### METHODS:

### 1] Synthesis of peptides.

All peptides (except peptide B: Fig. 1) were synthesized as C-terminal amides on a Rink resin (loading 0.38 mmol/g), using the FastMoc^{™} method (7) on an ABI 430A peptide synthesizer. Cysteine sulphur was protected with a S-tert-butylsulfenyl group or a trityl group, all other side chain functions were protected with trifluoroacetic acid labile groups. After completion of the synthesis, peptides were either acetylated using acetic anhydride/DIEA/NMP (0.1/0.01/1 (v/v/v)) or N-palmitoylated using pre-activated palmitic acid (see Methods; sections 3 and 4). Peptides with S-trityl protected cysteine were cleaved from the resin and concomitantly deprotected by acidolysis in the presence of scavengers using TFA/water/EDT/TA/phenol (40/2/1/2/3) (v/v/v/v/w)) for 2 hours. The crude peptides were precipitated and washed twice with hexane/ether (1:1) and lyophilized from acetonitrile/water (1:1). Peptide B was synthesized as C-terminal amide on a methylbenzhydrylamine (MHBA, 0.46 mmol/g) resin, starting with Fmoc-Lys(Boc)-OH in NMP. After cleavage of the Boc group by TFA/water (19:1), lysine was palmitoylated with pre-activated palmitic acid (see Methods; section 4). After Fmoc deprotection, the α-amino groups were successively acylated as described above with the pertinent Fmoc-amino acids to give the protected peptide. Compound B was deprotected and cleaved from the resin by HF/anisole (9:1).

### 2] Deprotection of S-(tert-butylsulfenyl) cysteinyl residues in solution.

The side chain protection of cysteine was removed using a reducing agent: a double deprotection of 30 minutes was routinely used, each with a 50 eq. molar excess tri(n-butyl)phosphine in 10% H₂O/NMP under nitrogen atmosphere. The reaction was monitored by HPLC analysis.

### 3] S-palmitoylation in solution.

- *Activation of palmitic acid.* To a solution of palmitic acid (100 µmol) in DCM (250 µl) was added 100 µmol of HBTU/HOBt in DMF (0.45M, 220 µl) and 200 µmol 2M DIEA in NMP. The activated palmitic acid formes a gel from this mixture in about 20 minutes at room temperature.
- *Palmitoylation of* a *thiol group.* An acetylated peptide (not containing lysyl residues) was dissolved in NMP at a concentration of 10 mg/ml. The solution was brought to pH 5 with DIEA (2M in NMP), as checked by using wet pH paper, and mixed with one eq. of pre-activated palmitic acid. After 90 min at room temperature and stirring, a sample was subjected to analytical HPLC. About 50% of the peptide was found to have reacted. A second equivalent of pre-activated palmitic acid, this time prepared with only half the amount of DIEA, was added and after another 90 min more than 70% of the peptide was palmitoylated and no by-products were found. After a total reaction period of 5 hours, the mixture was diluted fourfold with acetonitrile/water (1:1), two drops of TFA were added and the palmitoylated peptide was isolated and purified by preparative HPLC.
- *Palmitoylation of an* α- or *an* ε-*amino group.* A free Nα or a free Nε of a lysyl residue were N-palmitoylated as decribed for S-palmitoylation.

### 4] S-palmitoylation on the resin.

After completion of the synthesis, the resin was treated twice for 60 minutes (or the second time overnight, depending on the quality of the phospine) with tri(n-butyl)phosphine (50 eq.) in NMP/water (9:1) to remove the tert-butylsulfenyl group. The resin was washed copiously with NMP. Free SH groups were palmitoylated on the resin by double coupling using the pre-activated palmitic acid reagent (see palmitoylation in solution, section 3) in DCM/NMP for 5, resp. 16 hours.

### 5] selection of deprotection mixture and stability of thioester with respect to acid

To investigate the stability of thioesters during acidolytic removal of tert-butyloxy functions (Boc and OBu^{t}) and/or cleavage of the resin-peptide bond, two model peptides, Ac-C(palm)SEIFRPGGGDMR-NH₂ and Ac-C(palm)VATQLPASF-NH₂, both not containing lysine, were palmitoylated in solution and purified with preparative HPLC. Samples were then subjected to the conditions of acidolysis in the presence or the absence of scavengers using TFA/water (19/1 v/v) (A), TFA/water/EDT/TA/phenol (40/2/1/2/3 v/v/v/v/w) (B), or EDT/water/TFA (1/1/38 v/v/v/) (C). Acetic acid/water (1/1) was used as a control. The treatment (3 hours) was at room temperature. The thioester proved to be stable under all these acidic conditions.

### 6] Blocking of free SH groups by iodoacetamide.

To prevent the peptide ac-CSDGAVQPDGGQPAVRNERATG-NH₂ from dimerization by air oxidation, the free SH group was blocked. Peptide ac-CSDGAVQPDGGQPAVRNERTAG-NH₂ (20 µmol) and iodoacetamide (100 µmol) were dissolved in 400 µl DMF and 600 µl 2% NH₄HCO₃ (pH 8.0). The pH was kept between 7.5 and 8.5, and if necessary corrected with extra NH₄HCO₃ (grains). The solution was stirred for some 1.5 or 2 hours at room temperature. Acetic acid (100%, 50 µl) was added to stop the reaction. An Ellmann test was used to analyse the result.

### 7] Stability of the thioester with respect to base.

The stability of peptide A was investigated in phosphate buffered saline (PBS, pH 7.2)/DMF (7/3), aqueous NH₄HCO₃ (2%, pH 8)/DMF (7/3) piperidine/NMP (3/7). Peptide A was dissolved at a concentration of 5 mg/ml, at room temperature. The stability was monitored by analytical HPLC (Table 1). The results are summarized in table 1.

### 8] Synthesis of_disulfide construct C of CPV

ac-CSDGAVQPDGGQPAVRNERATG-NH₂ (13 mg) and palm-CSDGAVQPDGGQPAVRNERATG-NH₂ (6.4 mg) were dissolved in 1 ml of 50% DMSO/DCM. The solution was brought to pH 7.0 with 150 µl of 2% NH₄HCO₃ The mixture was stirred and the reaction was followed by analytical HPLC. After 3 hours the reaction was stopped by the addition of 0.5 ml of acetic acid. The heterodimer C (Fig. 1, disulfide) and the homodimer of the acetyl peptide were isolated by HPLC purification. To obtain an optimal yield of compound C equimolar quantities of both starting peptides can be used. The dimerization can also be performed in 10% DMSO/NMP using triethylamine for neutralization. The molar mass was determined by FAB-MS. Since reducing thioglycolic acid was added for mass analysis, only the two single chain peptides were found (ac-peptide: calc: 2422, found 2422 and 2489; palm-peptide: calc: 2465.79, found 2465,20). Two side reactions, that occured during synthesis, were responsible for a number of side products (26): an extra mass of 67 was caused by a reaction of piperidine with one of the aspartic acid residues, losing one molecule of water, and a loss of 18 was caused by an Asp-Gly rearrangement (DG occurs twice in the sequence!), a side reaction that frequently occurs especially in DG sequences. Electrospray MS (ES-MS) revealed the mass of the complete construct C, but also the masses of the construct C modified by the side reactions mentioned above. These modifications will not influence the immuneresponse significantly.

### 9] Conjugate preparation.

Keyhole limpet haemocyanin (KLH, 10 mg, Calbiochem, La Jolla, CA) was dissolved in 0.1 M sodiumphosphate buffer, pH 7.0, at a concentration of 12.5 mg/ml. Acetonitrile (0.3 ml) was added, followed by 100 µl of 0.125 M maleimidobenzoyl-N-hydroxysuccinimide ester (MBS, Pierce, Rockford, IL) solution in DMF. After stirring for 1 hour, the solution was dialysed three times for 30 minutes against 0.1 M phosphate buffer, pH 7.0 at 4°C. Peptide (10 mg) was added to the modified KLH and the mixture was shaken overnight at room temperature. The conjugate was dialyzed two times for 2 hours and then overnight against 0.1 M phosphate buffer, pH 7.0 at 4°C.

### 10] Vaccine formulation.

For each vaccine, peptide constructs (conjugated to KLH, N- or S-palmitoylated) were dissolved in phosphate-buffered saline (PBS, pH 7.2) and emulsified with complete Freund's adjuvant (CFA) in equal volumes. Mixtures were emulsified by repeatedly forcing it through a needle until a stable water-in-oil emulsion was obtained. The emulsion was prepared just before immunization. Second immunizations were prepared with incomplete Freund's adjuvant (IFA).

### 11] Vaccination or immunization.

*GnRH-tandem constructs.* Six groups of 7-8 male piglets at the age of 10 weeks were immunized with compound A or B (Fig. 1). Each animal was injected with 1 ml of vaccine formulation (1, 0.25 or 0.05 mg) administered intramuscularly in the neck. The second immunization was given 8 weeks later. One group served as a control and was mock immunized with only CFA and KLH. At week 26 the animals reached their slaughterweight; their testicles were excised, epididymes were removed and testes weight was recorded.

*CPV constructs.* In the first experiment, two groups of 5 guinea pigs were immunized with compound C or D (Fig. 1). Each animal was injected with 500 µl vaccine formulation administered subcutaneously in the neck. The second immunization was given 6 weeks later. One group served as a control and was immunized with compound F (KLH-MBS coupled peptide construct, Fig. 1). Blood was taken at weeks 0, 6, 7 and 12 post immunization and anti peptide antibody titers were recorded in an ELISA.

In the second experiment, one group of 3 guinea pigs was immunized with compound C emulsified in CFA. For comparison, compound E was emulsified in CFA. Blood was taken at 0, 4, 8 and 16 weeks post immunization and anti peptide antibody titers were recorded in an ELISA.

In the third experiment, three groups of three guinea pigs were immunized with compound D, E or F. CFA was used to emulsify the peptides. As a control, the free peptide G was used in which the thiol group was blocked with iodoacetamide to prevent dimerization of the peptide. Blood was taken at 0, 4 and 8 weeks post immunization and anti peptide antibody titers were recorded in an ELISA.

*FIV constructs.* In this experiment six groups of four cats were immunized with compound H (in IFA or in ISCOMS) or with compound I (conjugated with cholera toxin via MBS, given either subcutaneously, rectally, or intranasally). Blood samples were taken at 8 weeks post vaccination and antibody titers against the peptide were determined in an ELISA.

### Results

### 1] Methodological.

A method has been developed for the acylation of peptide thiols in solution or within the matrix of the solid support. In solution, peptides with only one single nucleophilic function (N or S) can be acylated in a very efficient way using pre-activated palmitic acid. S-acylation of protected peptides on the solid support requires an efficient and selective removal of the *tert-*butylsulfenyl protection, which was realized effectively by reduction with tri(n-butyl)phosphine in NMP/water (9/1). An advantage of this approach is that it can be done in the presence of lysines, because the lysines are still protected. The thiol palmitates (A and E; Fig. 1) were obtained by acylation in solution with a pre-activated palmitic acid; B, E and the palmitoyl peptide of compound C were palmitoylated on the resin. Thioester peptides were found to be stable during acidolysis of other protective functions. The palmitoyl-thioester bond is not stable under basic conditions. Buffered solutions of the thioesters around pH 8 should not be left standing for prolonged periods (Table 1).
At pH 7, stability is already much better and lyophilized thioester at -18°C was found to be completely stable after 28 months.

### 2] Immunogenic potency of palmitoylated peptides.

### a] GnRH-tandem peptides A and B:

The immunogenic potency of the synthetic GnRH tandem peptides A and B was tested in an experiment on six groups of 7-8 male piglets (age 10 weeks), each group recieving a dose of either S-palmitoylated (A) or N-palmitoylated (B) antigen. The GnRH tandem peptides A and B exhibited different immunogenic effects, as observed on testis weight (15) at the age of 26 weeks after two injections in the presence of CFA or IFA (second injection, see Table 2). While both forms were able to keep testis size low, only the S-palmitoylated peptide A was able to do this in all animals at the highest dose tested (1 mg), and still in 3 out *of 8 animals at the lowest dose (0.05 mg) tested. The response was also dose dependent. A dose of 0.25 mg S-palmitoylated peptide A gave a similar effect as a dose of 1 mg N-palmitoylated peptide B.

### b] CPV peptides:

The above observed effect with the palmitoylated GnRH peptides A and B was further investigated with another construct, and in another animal species. In following experiments, the N-ternimal fragment of VP2 (positions 2-21) of canine parvovirus was used. This peptide, when conjugated via a disulfide bridge to the same peptide with a N-palmitoyl group (peptide construct C), appeared to be capable to elicit antibody responses as well as the peptide conjugated to KLH (peptide F, etc.). On the other hand, the N-palmitoylated peptide alone (peptide D, probably partly present as dimer) did yield a lower immunogenic effect, since antibody titers were lower and did not develop in all animals (see Table 3). Peptide construct C was formed during first synthesis of peptide E. In this synthesis we coupled Fmoc-Cys(StBu)-OH to the parvo peptide-resin, then cleaved the StBu side chain protecting group of cysteine, using 2-mercaptoethanol for 48 hours. This step also partially cleaved the Fmoc group. The resin was washed and the free cysteine was palmitoylated at the thiol group and partially at the amino group as described. Next, the Fmoc group was cleaved using piperidine. In this step the palmitoyl thioester was also cleaved and partially shifted to the free amino group of cysteine (S⇒N shift). Then, the peptide was acetylated and the peptide was deprotected and cleaved from the resin as described. After workup and purification by preparative HPLC, product C, a synthetic peptide linked with a disulfide bond to a fatty acid linked to another copy of said synthetic peptide, was isolated together with some minor side products. Mass spectrometry showed that peptide construct C was partially rearranged (26), because of the Asp-Gly sequence that occurs twice in the parvo peptide. However, the addition of a piperidyl group to aspartic acid at position 3 or 9, or the loss of water molecule by the formation of an aspartimide will not influence the immune response significantly. A second experiment was performed in which peptide E was compared with peptide construct C in CFA. In this synthesis of peptide E, the peptide was acetylated directly after the coupling of Fmoc-Cys(StBu)-OH, thus preventing palmitoylation of the amino group. After cleavage of the StBu group, S-palmitoylation, and final deprotection, the parvo thioester product was purified using HPLC. This resulted in a product E, a synthetic peptide linked with a thioester bond to a fatty acid, where no piperidine adducts were detected by analytical HPLC and mass analysis. Product E and product C had a similar immune response (see table 4). The superiority of the S-palmitoylated product above the N-palmitoylated peptide was further confirmed by direct comparison of peptide D, peptide E, and the KLH-MBS-conjugated peptide F (Table 5). In this third *experiment* the antibody titer elicited with the S-palmitoylated peptide at 4 weeks past vaccination was almost 100 times higher than the titer obtained with the N-palmitoylated peptide. At week 8 the titers were almost equal. Compared to the KLH-conjugated peptide, the titers for the S-palmitoylated peptide were both in the 4^{th} and the 8^{th} week higher.

### C] FIV peptides:

The antibody response of the palmitoyl-thioester peptide H, either in IFA or in ISCOMS was much higher than the response against the cholera toxin conjugated peptide. The way the CT peptide conjugate was administered could not improve the responses much (see Table 6).

### Discussion of experimental results in the examples

### Immunogenic aspects.

In this study, procedures for palmitoylation through an amide or thioester bond were established. Furthermore, we investigated the immunogenic potency of peptides conjugated to a fatty acid, and compared it with KLH-MBS-conjugated peptides. It was found that the site of acylation (N or S) effects the immunogenicity of the adduct. Surprisingly, palmitoylation of the peptide through a thioester bond (S acylation) yields a highly immunogenic product, being as potent as or even better than a peptide coupled to a carrier protein (KLH, OVA or CT). On the other hand, N-palmitoylation is far less immunogenic compared to S-palmitoylation or KLH-MBS conjugation. A peptide conjugated via a labile disulfide bond with an N-palmitoylated peptide carrier, however, also induces high antibody responses. Thus, a reversible and labile bond between the antigenic peptide and a carrier molecule, being palmitic acid or a palmitoylated peptide, drastically enhances the immune response. The introduction of a fatty acid through a thioester bond thus obviates the necessity of a large carrier protein. The conventional conjugation with a large molecule (a protein) induces difficulties because the chemistry underlying the coupling is not always well controlled, while in addition unwanted antibodies against carrier protein and linker are induced.

### Chemical aspects:

Nα-acetylated peptide amides can be prepared automatically through solid-phase synthesis. However, removal of the base-labile Fmoc groups with piperidine precludes the use of S-palmitoylated cysteinyl derivatives during synthesis. Therefore, S-acylation had to be performed at the end of the synthesis on the resin or after cleavage and deprotection in solution. The acylation reaction applied here can not discriminate between thiols and amino groups. Since peptides upon cleavage from their support loose also their N-protecting groups, selective palmitoylation can only be performed in solution with compounds comprising one single nucleophilic function (N or S). Hydroxyl groups are not affected.

Because of the instability with respect to base it is not recommended to leave S-palmitoylated peptides in buffered (pH 7-8) solutions for prolonged periods (see Table 1). Thioesters do not dissociate in acidic media and do thus not contain thiolate anions which would attack thioesters. We confirm the general rule that thioesters resist thiols in strongly acidic media enabling the use of thiols as scavengers for carbonium ions during deprotection of tert-butyloxy functions (cf. 21).

### References

1. Albert, B., D. Bray, J. Lewis, M. Raff, K. Roberts and J.D. Watson. 1983. Molecular Biology of the Cell, Garland Publishing, Inc., New York & London, page 333.
2. Bodanszky, M. 1984. Principles of peptide synthesis, p. 132. In K. Hafner, J.-M. Lehm, C.W. Rees, P. von Rague Schleyer, B.M. Trost and R. Zahradnik (Eds.), Reactivity and structure: Concepts in organic chemistry, vol 16. 1984. Springer-Verlag Berlin.
3. Browning, M., G. Reid, R. Osborne and O. Jarrett. 1992. Incorporation of soluble antigens into ISCOMs: HIV gp120 ISCOMs induce virus neutralizing antibodies. Vaccine 10 (9) : 585-590 .
4. Defoort, J.-P., B. Nardelli, W. Huang, D.R. Shia and J.P. Tam. 1990. Complete synthetic vaccine with built-in adjuvant, p. 845-846. In E. Giralt and D. Andreu (Eds.) Peptides 1991. ESCOM, Leiden.
5. Deprez, B., H. Gras-Masse, F. Martinon, E. Gomard, J.P. Levy and A. Tartar. 1994. Synthetis and evaluation of novel lipopeptide constructs for in vivo induction of virus-specific cytotoxic T-lymphocyte mediated response, p. 736-738. In R.S. Hodges & J.A. Smith (Eds.), Peptides, Chemistry, Structure and Biology 1994. ESCOM, Leiden.
6. Deschenes, R.J., M.D. Resh and J.R. Broach. 1990. Acylation and prenylation of proteins. Current opinions in cell biology 2:1108-1113.
7. Fields, C.G., D.H. Lloyd, R.L. Macdonald, K.M. Otteson and R.L. Noble. 1991. HBTU activation for automated Fmoc Solid Phase Peptide Synthesis. Peptide Research 4(2):95-101.
8. Hopp, T.P. 1984. Immunogenicity of a synthetic HBsAg peptide: enhancement by conjugation to a fatty acid carrier. Molecular Immunology 21(1):13-16.
9. Huang, W., B. Nardelli, D.R. Shia and J.P. Tam. 1990. Synthetic vaccine mimetic, p. 847-848. In E. Giralt and D. Andreu (Eds.), Peptides 1991. ESCOM, Leiden.
10. Huang, W., B. Nardelli and J.P. Tam. 1994. Lipophilic multiple antigen peptide system for peptide immunogen and synthetic vaccine. Molecular Immunology 31 (15):1191-1199.
11. Langeveld, J.P.M., J.I. Casal, E. Cortes, G. van de Wetering, R.S. Boshuizen, W.M.M. Schaaper, K. Dalsgaard and R.H. Meloen. 1994. Effective induction of neutralizing antibodies with the amino terminus of VP2 of canine parvovirus as a synthetic peptide. Vaccine 12(15):1473-1479.
12. Langeveld, J.P.M., S. Kamstrup, A. Uttenthal, B. Strandbygaard, C. Vela, K. Dalsgaard, N.J.C.M. Beekman, R.H. Meloen and I. Casal. 1995. Full protection in mink against mink enteritis virus with new generation canine parvovirus vaccines based on synthetic peptide or recombinant protein. Vaccine 13(10):1033-1037.
13. Metzger, J., K.-H. Wiesmuller, R. Schaude, W.G. Bessler and G. Jung. 1991. Synthesis of novel immunologically active tripalmitoyl-S-glycerylcysteinyl lipopeptides as useful intermediates for immunogen preparations. Int. J. Peptide Protein Res. 37:46-57.
14. Mowat, M.I., A.M. Donachie, G. Reid and O. Jarrett. 1991. Immune-stimulating complexes containing Quil-A and protein antigen prime class I MHC-restricted T lymphocytes in vivo and are immunogenic by the oral route. Immunology 72:317-322.
15. Oonk, R.B., J.A. Turkstra, H. Lankhof, W.M.M. Schaaper, J.H.M. Verheyden, and R.H. Meloen. 1995. Testis size after immunocastration as parameter for the absence of boar taint. Lifestock Production Science 42:63-71.
16. Peitzsch, R.M. and McLaughlin, S. 1993. Binding of acylated peptides and fatty acids to phospholilid vesicles: pertinence to myristoylated proteins. Biochemistry 32:10436-10443
17. Reid, G. 1992. Soluble proteins incorporate into ISCOMs after covalent attachment of fatty acid. Vaccine 10 (9) :597-602.
18. Resh, M.D. 1994. Myristylation and palmitylation of Src family members: the fats of the matter. Cell 76:411-413
19. Rink, H. 1987. Solid-phase synthesis of protected peptide fragments using a trialkoxy-diphenyl-methyl ester resin. Tetrahedron Letters 28:3787-3790.
20. Robinson, J.H., M.C. Case and C.G. Brooks. 1992. Palmitic acid conjugation of a protein antigen enhances major histocompatibility complex class II-restricted presentation to T cells. Immunology 76:593-598.
21. Seidel, C., C. Klein, B. Empl, H. Bayer, M. Lin, and H.-G. Batz. 1991. Synthesis of a cyclic peptide for diagnostic use by oxidation of its resin-bound intermediate, p. 236-237. In J.A. Smith and J.E. Rivier (Eds.), Peptides. Chemistry and Biology. 1992. ESCOM, Leiden.
22. Stryer, L. 1981. Biochemistry, second edition. Eds: W.H. Freeman and company, San Fransisco, page 661.
23. Toth, I. 1994. A novel chemical approach to drug delivery: lipidic amino acid conjugates. Journal of drug targeting 2:217-239.
24. Wang, C.Y., D.J. Looney, M.L. Li, A.M. Walfield, J. Ye, B. Hosein, J.P. Tam and F. Wong-Staal. 1991. Long-term higher-titer neutralizing activity induced by octameric synthetic HIV-1 antigen. Science 254:285-288.
25. Wedegaertner, P.B. and Henry R. Bourne. 1994. Activation and depalmitoylation of GSα. Cell 77:1063-1070.
26. Tam, J.P. 1994. Molecular Immunology 31:1191-1199.
27. Yang, Y., W.V. Sweeney, K. Schneider, S. Thornqvist, B.T. Chait, and J.P. Tam. 1994. Aspartimide formation in base-driven 9-Fluorenylmethoxycarbonyl chemistry. Tetrahedron Letters 35: 9689-9692.

**Table 1. stability of the thioester bond in basic solutions**

| | 2 hours | 22 hours | 95 hours |
|---|---|---|---|
| PBS(pH 7)/DMF (7:3) | >99% | 90% | 62% |
| 2%NH₄HCO₃(pH8)/DMF (7:3) | >97% | 30% | 0% |
| piperidine/NMP (3:7) | 0% | 0% | 0% |

| | | | |
|---|---|---|---|
| Results are given as percentage of the initial concentration of GnRH tandem peptide (A) as estimated by analytical HPLC after a given time period at room temperature. | | | |

**Table 2. Testis weight of male piglets immunized with different doses of GnRH tandem peptides**

| peptide | dose (µg) | testis weight^{a} | | |
|---|---|---|---|---|
| | | <100 | 100-150 | >150 |
| A (thioester) | 1000 | 7/7 | 0/7 | 0/7 |
| " | 250 | 6/8 | 1/8 | 1/8 |
| " | 50 | 3/8 | 0/8 | 5/8 |
| | | | | |
| B (amide) | 1000 | 5/7 | 0/7 | 2/7 |
| " | 250 | 1/7 | 2/7 | 4/7 |
| " | 50 | 0/6 | 0/6 | 6/6 |
| | | | | |
| control (CFA) | - | 0/7 | 0/7 | 7/7 |

| | | | | |
|---|---|---|---|---|
| ^{a}: number of animals in weight category/total number of animals. Testis weights <100 gram were considered positive, between 100-150 gram intermediate and more than 150 gram negative. | | | | |

**Table 3: Serum anti-peptide antibody titers at 6, 7 and 12 weeks of guinea pigs vaccinated with 100 µg compound C, D or F emulsified in CFA. Booster vaccination emulsified in IFA was given at week 6 post first vaccination. Blood samples were already taken.**

| | | | titer^{a} | |
|---|---|---|---|---|
| animal | compound | 6wpv | 7wpv | 12wpv |
| 1 | C | 5.1 | 6.0 | >6.5 |
| 2 | C | 5.1 | 5.0 | 4.8 |
| 3 | C | 6.1 | 5.4 | 5.4 |
| 4 | C | 5.0 | 4.9 | 5.0 |
| 5 | C | 4.6 | #^{b} | |
| 6 | D | 3.5 | 4.1 | 4.3 |
| | | | | |
| 7 | D | 1.7 | 1.6 | 2.9 |
| 8 | D | 3.5 | 3.7 | 3.7 |
| 9 | D | -^{c} | - | - |
| 10 | D | 2.0 | 2.0 | # |
| | | | | |
| 11 | F | 5.1 | 5.3 | 5.7 |
| 12 | F | 5.0 | 5.0 | >6.5 |
| 13 | F | 4.5 | 4.7 | # |
| 14 | F | 5.2 | # | |
| 15 | F | 5.0 | 5.2 | 5.3 |

| | | | | |
|---|---|---|---|---|
| ^{a}: zero blood samples were subtracted before calculation. The titer was calculated as the -log(dilution factor) which give a signal three times the background. ^{b}: animal died during the experiment. ^{c}_{:} means no detectable titer, lowest dilution measured was 1/10 | | | | |

**Table 4: Serum anti-peptide antibody titers after 4, 8 and 16 weeks in guinea pigs. Compound C was compared with compound E, both emulsified in CFA.**

| | | | titer^{a} | |
|---|---|---|---|---|
| animal | compound | 4wpv | 8wpv | 16wpv |
| 1 | C | 2.2 | 3.7 | 3.8 |
| 2 | C | 2.1 | 2.4 | 2.8 |
| 3 | C | 2.2 | 3.8 | 3.8 |
| 4 | E | -^{b} | 3.0 | 4.0 |
| 5 | E | 2.1 | 3.5 | 4.6 |
| 6 | E | 2.1 | 2.4 | 2.5 |

| | | | | |
|---|---|---|---|---|
| ^{a}: zero blood samples were subtracted before calculation. The titer was calculated as the -log(dilution factor) which give a signal three times the background. ^{b}_{:} - means titer lower than 2.0 | | | | |

**Table 5: Serum anti-peptide antibody titers after 4 and 8 weeks of guinea pigs immunized with 100 µg palmitoylated peptides (S or N), conjugated (KLH-MBS) or blocked (iodoacetamide).**

| | | titer^{a} | |
|---|---|---|---|
| animal | compound | 4 wpv | 8 wpv |
| 1 | E | 2.5 | 2.5 |
| 2 | E | 4.0 | 3.3 |
| 3 | E | 3.1 | 3.4 |
| | | | |
| 4 | D | 1.8 | 2.9 |
| 5 | D | -b | 3.4 |
| 6 | D | - | 2.3 |
| | | | |
| 7 | F | 2.5 | 2.2 |
| 8 | F | 2.7 | 2.0 |
| 9 | F | - | - |
| | | | |
| 10 | G | - | - |
| 11 | G | - | - |
| 12 | G | - | - |

| | | | |
|---|---|---|---|
| ^{a}: zero blood samples were subtracted before calculation of the titer. The titer was calculated as the - log(dilution factor) which give a signal three times the background. ^{b}: - no detectable titer, lowest dilution measured was 1/30. | | | |

**Table 6: Serum anti-peptide antibody titers in cats.**

| animal | compound | titer^{a} |
|---|---|---|
| 1 | H/IFA | 1.60 |
| 2 | H/IFA | 0.85 |
| 3 | H/IFA | 1.70 |
| 4 | H/IFA | 1.35 |
| 5. | H/ISCOM | 0.20 |
| 6 | H/ISCOM | 1.35 |
| 7 | H/ISCOM | 0.07 |
| 8 | H/ISCOM | 1.25 |
| 9 | I/CT-SC | 0.15 |
| 10 | I/CT-SC | 0.10 |
| 11 | I/CT-SC | 0.12 |
| 12 | I/CT-SC | 0.02 |
| 13 | I/CT-R | 0.06 |
| 14 | I-CT-R | 0.01 |
| 15 | I/CT-R | 0 |
| 16 | I/CT-R | 0 |
| 17 | I/CT-IN | 0.02 |
| 18 | I/CT-IN | 0.10 |
| 19 | I/CT-IN | 0.01 |
| 20 | I/CT-IN | 0.15 |
| 21 | CONTROL | 0 |
| 22 | CONTROL | 0 |
| 23 | CONTROL | 0 |
| 24 | CONTROL | 0 |

| | | |
|---|---|---|
| ^{a} titers as optical density minus background | | |

## Claims

1. A vaccine comprising an antigen and a fatty acid or fatty acid peptide carrier compound which are directly or indirectly linked by a thioester bond that is labile and dissociates under certain physiological conditions.

2. A vaccine according to claim 1 in which said antigen dissociates from said carrier compound after the vaccine or preparation has been administered.

3. A vaccine according to claim 1 or 2 in which said antigen is a protein, a polypeptide, a synthetic peptide, a carbohydrate, or a hapten.

4. A vaccine according to claim 3 in which the antigen is a synthetic peptide.

5. A vaccine according to any one of claims 1-4 in which the fatty acid is palmitic acid.

6. A vaccine according to claim 4 or 5 in which the synthetic peptide essentially consists of the amino acid sequence EHWSYGLRPGQHWSYGLRPG.

7. A vaccine according to claim 4 or 5 in which the synthetic peptide essentially consists of the amino acid sequence SDGAVQPDGGQPAVRNERATG.

8. A vaccine according to claim 4 or 5 in which the synthetic peptide essentially consists of the amino acid sequence RAISSWKQRNRWEWPRD.

9. A vaccine according to any one of claims 1-4 in which the antigen is a peptide and the carrier compound is another copy of said peptide coupled to a fatty acid.

10. A vaccine according to claim 9 in which the carrier compound is an N-palmitoylated peptide.

11. A vaccine according to claim 9 or 10 in which the peptide essentially consists of the amino acid sequence SDGAVQPDGGQPAVRNERATG.

12. A vaccine preparation according to any of claims 1-11 together with pharmaceutically acceptable compound or adjuvant.

13. A composition comprising a synthetic peptide linked with a thioester bond that is labile and dissociates under certain physiological conditions, to a fatty acid.

14. A composition according to claim 13 wherein the fatty acid is palmitic acid.

15. A composition according to claim 13 or 14 wherein the peptide is selected from the group consisting of EHWSYGLRPGQHWSYGLRPG, SDGAVQPDGGQPAVRNERATG and RAISSWKQRNRWEWPRD.

16. A method for producing an immunogenic preparation comprising linking a synthetic peptide with a fatty acid or fatty acid peptide carrier compound via a thioester bond that is labile and dissociates under physiological conditions.

17. A method according to claim 16 wherein the fatty acid is palmitic acid.

18. A method according to claim 16 or 17 wherein the peptide is selected from the group consisting of EHWSYGLRPGQHWSYGLRPG, SDGAVQPDGGQPAVRNERATG and RAISSWKQRNRWEWPRD.

19. An immunogenic preparation comprising a synthetic peptide and a fatty acid or fatty acid peptide carrier compound which are linked by a thioester bond that is labile and dissociates under certain physiological conditions.

20. A vaccine comprising an immunogenic preparation according to claim 19 together with pharmaceutically acceptable compound or adjuvant.

## Patentansprüche

1. Impfstoff, welcher ein Antigen und eine Fettsäure- oder Fettsäure-Peptid-Trägerverbindung umfasst, die direkt oder indirekt durch eine Thioesterbindung verbunden sind, welche labil ist und unter bestimmten physiologischen Bedingungen dissoziiert.

2. Impfstoff nach Anspruch 1, bei welchem das Antigen von der Trägerverbindung dissoziiert, nachdem der Impfstoff oder das Präparat verabreicht worden sind.

3. Impfstoff nach Anspruch 1 oder 2, bei welchem das Antigen ein Protein, ein Polypeptid, ein synthetisches Peptid, ein Kohlenhydrat oder ein Hapten ist.

4. Impfstoff nach Anspruch 3, bei welchem das Antigen ein synthetisches Peptid ist.

5. Impfstoff nach einem der Ansprüche 1 - 4, bei welchem die Fettsäure Palmitinsäure ist.

6. Impfstoff nach Anspruch 4 oder 5, bei welchem das synthetische Peptid im Wesentlichen aus der Aminosäuresequenz EHWSYGLRPGQHWSYGLRPG besteht.

7. Impfstoff nach Anspruch 4 oder 5, bei welchem das synthetische Peptid im Wesentlichen aus der Aminosäuresequenz SDGAVQPDGGQPAVRNERATG besteht.

8. Impfstoff nach Anspruch 4 oder 5, bei welchem das synthetische Peptid im Wesentlichen aus der Aminosäuresequenz RAISSWKQRNRWEWPRD besteht.

9. Impfstoff nach einem der Ansprüche 1 - 4, bei welchem das Antigen ein Peptid ist und die Trägerverbindung eine weitere Kopie des Peptids gekoppelt an eine Fettsäure darstellt.

10. Impfstoff nach Anspruch 9, bei welchem die Trägerverbindung ein N-Palmitoylpeptid ist.

11. Impfstoff nach Anspruch 9 oder 10, bei welchem das Peptid im Wesentlichen aus der Aminosäuresequenz SDGAVQPDGGQPAVRNERATG besteht.

12. Impfstoffpräparat nach einem der Ansprüche 1 - 11 zusammen mit einer pharmazeutisch akzeptablen Verbindung oder einem Adjuvans.

13. Zusammensetzung, welche ein synthetisches Peptid umfasst, das durch eine Thioesterbindung, welche labil ist und unter bestimmten physiologischen Bedingungen dissoziiert, an eine Fettsäure gebunden ist.

14. Zusammensetzung nach Anspruch 13, bei welcher die Fettsäure Palmitinsäure ist.

15. Zusammensetzung nach Anspruch 13 oder 14, bei welcher das Peptid aus einer Gruppe ausgewählt ist, die aus EHWSYGLRPGQHWSYGLRPG, SDGAVQPDGGQPAVRNERATG und RAISSWKQRNRWEWPRD besteht.

16. Verfahren zur Herstellung eines immunogenen Präparats, welches das Binden eines synthetischen Peptids an eine Fettsäure- oder Fettsäure-Peptid-Trägerverbindung über eine Thioesterbindung umfasst, die labil ist und unter physiologischen Bedingungen dissoziiert.

17. Verfahren nach Anspruch 16, bei welchem die Fettsäure Palmitinsäure ist.

18. Verfahren nach Anspruch 16 oder 17, bei welchem das Peptid aus einer Gruppe ausgewählt wird, die aus EHWSYGLRPGQHWSYGLRPG, SDGAVQPDGGQPAVRNERATG und RAISSWKQRNRWEWPRD besteht.

19. Immunogenes Präparat, welches ein synthetisches Peptid und eine Fettsäure- oder Fettsäure-Peptid-Trägerverbindung umfasst, die durch eine Thioesterbindung verbunden sind, welche labil ist und unter bestimmten physiologischen Bedingungen dissoziiert.

20. Impfstoff, der ein immunogenes Präparat gemäß Anspruch 19 zusammen mit einer pharmazeutisch akzeptablen Verbindung oder einem Adjuvans umfasst.

## Revendications

1. Vaccin comprenant un antigène et un composé support acide gras ou peptide acide gras qui sont directement ou indirectement liés par une liaison thioester qui est labile et se dissocie dans certaines conditions physiologiques.

2. Vaccin selon la revendication 1 dans lequel ledit antigène se dissocie dudit composé support après l'administration du vaccin ou de la préparation.

3. Vaccin selon la revendication 1 ou 2 dans lequel ledit antigène est une protéine, un polypeptide, un peptide synthétique, un glucide ou un haptène.

4. Vaccin selon la revendication 3 dans lequel l'antigène est un peptide synthétique.

5. Vaccin selon l'une quelconque des revendications 1 à 4 dans lequel l'acide gras est l'acide palmitique.

6. Vaccin selon la revendication 4 ou 5 dans lequel le peptide synthétique se compose essentiellement de la séquence d'acides aminés EHWSYGLRPGQHWSYGLRPG.

7. Vaccin selon la revendication 4 ou 5 dans lequel le peptide synthétique se compose essentiellement de la séquence d'acides aminés SDGAVQPDGGQPAVRNERATG.

8. Vaccin selon la revendication 4 ou 5 dans lequel le peptide synthétique se compose essentiellement de la séquence d'acides aminés RAISSWKQRNRWEWPRD.

9. Vaccin selon l'une quelconque des revendications 1 à 4 dans lequel l'antigène est un peptide et le composé support est une autre copie dudit peptide couplée à un acide gras.

10. Vaccin selon la revendication 9 dans lequel le composé support est un peptide N-palmitoylé.

11. Vaccin selon la revendication 9 ou 10 dans lequel le peptide se compose essentiellement de la séquence d'acides aminés SDGAVQPDGGQ PAVRNERATG.

12. Préparation vaccinale selon l'une quelconque des revendications 1 à 11 ainsi qu'un composé ou adjuvant pharmaceutiquement acceptable.

13. Composition comprenant un peptide synthétique lié, par une liaison thioester qui est labile et se dissocie dans certaines conditions physiologiques, à un acide gras.

14. Composition selon la revendication 13 dans laquelle l'acide gras est l'acide palmitique.

15. Composition selon la revendication 13 ou 14 dans laquelle le peptide est choisi parmi le groupe constitué de EHWSYGLRPGQHW YGLRPG, SDGAVQPDGGQPAVRNERATG et RAISSWKQRRWEWPRD.

16. Procédé pour produire une préparation immunogène consistant à lier un peptide synthétique à un composé support acide gras ou peptide acide gras via une liaison thioester qui est labile et se dissocie dans des conditions physiologiques.

17. Procédé selon la revendication 16 dans lequel l'acide gras est l'acide palmitique.

18. Procédé selon la revendication 16 ou 17 dans lequel le peptide est choisi parmi le groupe constitué de EHWSYGLRPGQHWSYGLRPG, SDGAVQPDGGQPAVRNERATG et RAISSWKQRNRWEWPRD.

19. Préparation immunogène comprenant un peptide synthétique et un composé support acide gras ou peptide acide gras qui sont liés par une liaison thioester qui est labile et se dissocie dans certaines conditions physiologiques.

20. Vaccin comprenant une préparation immunogène selon la revendication 19 ainsi qu'un composé ou adjuvant pharmaceutiquement acceptable.
